# EUROPÄISCHE PATENTSCHRIFT

(11) **EP 2 456 760 B1**
(45) Veröffentlichungstag und Bekanntmachung des Hinweises auf die Patenterteilung: **07.05.2014**
(21) Anmeldenummer: 10734918.5
(22) Anmeldetag: 14.07.2010
(51) Int. Cl.: C07D 231/08, C07D 231/12

(54) **VERFAHREN ZUM HERSTELLEN VON ARYL-SUBSTITUIERTEN PYRAZOLEN**
Method for manufacturing aryl-substituted pyrazoles
Procédé de fabrication de pyrazoles substitués par de l'aryle

(30) Priorität: 23.07.2009 EP 09166238
(43) Veröffentlichungstag der Anmeldung: 30.05.2012
(73) Patentinhaber: Bayer CropScience AG, 40789 Monheim (DE)
(72) Erfinder: PAZENOK, Sergii, 42699 Solingen (DE); LUI, Norbert, 51519 Odenthal (DE)
(86) Internationale Anmeldenummer: PCT/EP2010/004285
(87) Internationale Veröffentlichungsnummer: WO 2011/009551

(56) Entgegenhaltungen:
- EP-A1- 0 659 745
- WO-A1-99/62885
- ALEX F.C. FLORES ET AL.: "Haloacetylated Enol Ethers,19:Synthesis of 3-(2-Thienyl)- and 3-(2-Furyl)-5-trihalomethyl Substituted Azoles" SYNTHESIS, Bd. 16, 4. August 2005 (2005-08-04), Seiten 2744-2750, XP002565006 Georg Thieme Verlag Stuttgart

## Beschreibung

Die vorliegende Erfindung betrifft ein Verfahren zur Herstellung von 1-(Aryl)-substituierten Pyrazolen umfassend die Umsetzung von Alkoxyenonen und Enaminoketonen mit Hydrazin-Derivaten zu 1-(Aryl)-substituierten dihydro-1H-Pyrazolen, deren Weiterreaktion unter Wasserabspaltung zu 1-(Aryl)-substituierten Trihalogenmethylpyrazolen und deren Weiterverarbeitung.

1-(Aryl)-substituierte Pyrazole und 1H-Pyrazole sind wertvolle Zwischenprodukte zur Herstellung von Anthranilsäureamiden, die als Insektizide Verwendung finden können.

In der Literatur ist bereits beschrieben, dass Pyrazole durch Reaktion von 1,3 Dicarbonylen oder entsprechenden 1,3 bis-elektrophilen Reagenzien mit Monoalkyl- oder Monoarylhydrazinen gebildet werden können (Synthesis 2004, N1. pp 43-52). Jedoch wird berichtet, dass im Fall von Monoalkyl- oder Monoarylhydrazinen eine Mischung aus regioisomeren Pyrazolen resultiert (Tetrahedron 59 (2003), 2197-2205; Martins et al., T. L. 45 (2004) 4935). Versuche, exklusiv ein Regioisomer zu erhalten, schlugen fehl (JOC 2007, 72822 8243-8250). In der Literatur ebenfalls beschrieben ist ein Verfahren zur Herstellung von Trifluormethyl-Pyrazolen (WO 2003/016282). Ebenfalls sind Herstellverfahren von (Het)Aryl-substituierten Pyrazolen beschrieben (WO 2007/144100), wobei durch Reduktion von Diestern mit DIBAL oder LiALH₄ die entsprechenden Pyrazole erhalten werden. Allerdings sind dabei sehr tiefe Temperaturen erforderlich, und die Verwendung von DIBAL ist unwirtschaftlich.

Die Aufgabe der vorliegenden Erfindung ist daher die Bereitstellung neuer, wirtschaftlicher Verfahren zur Herstellung von 1-(Aryl)-substituierten Pyrazol-Derivaten und 1-(Aryl) -substituierten dihydro-1H-Pyrazolen, die die zuvor beschriebenen Nachteile nicht aufweisen und welche sich durch eine auch im großtechnischen Maßstab besonders gut und einfach durchzuführende Prozeßführung auszeichnen.

Die Aufgabe wurde gemäß der vorliegenden Erfindung gelöst durch ein Verfahren zur Herstellung von 1-(Aryl)-substituierten-Pyrazol-Derivaten der allgemeinen Formel (I) 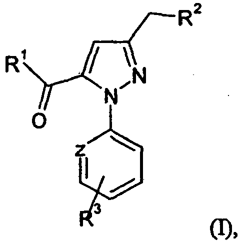 in welcher
- R¹: für Hydroxy, Alkoxy, Aryloxy, Halogen steht,
- R¹: bevorzugt für Hydroxy, (C₁-C₆)Alkoxy, Halogen steht,
- R¹: besonders bevorzugt für Hydroxy, (C₁-C₆)Alkoxy steht,
- R²: für Hydroxy, Alkoxy, Arylalkoxy, Alkylthio, Halogen, O-(C=O)Alkyl, O-(C=O)O-Alkyl, (C=O)Halogenalkyl, OSO₂Alkyl, OSO₂-Halogenalkyl, OSO₂-Aryl steht,
- R²: bevorzugt für Hydroxy, Halogen, O-(C=O)(C₁-C₆)Alkyl steht,
- R²: besonders bevorzugt für Hydroxy, O(C=O)CH₃ steht,
- R³: für H, Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
- R³: bevorzugt für H, Halogen, CN, NO₂, (C₁-C₆)-Alkyl, Halogen(C₁-C₆)-alkyl, (C₁-C₆)Alkoxy, Halogen(C₁-C₆)alkoxy, steht,
- R³: besonders bevorzugt für F, Chlor, Brom, Jod, CN, (C₁-C₄)-Alkyl, Halogen(C₁-C₄)-alkyl, Halogen(C₁-C₄)alkoxy, steht,
- R³: ganz besonders bevorzugt für Fluor, Chlor, Brom, Jod steht,
- R³: insbesondere bevorzugt für Chlor steht,
- Z: für CH, N steht,
- Z: bevorzugt und besonders bevorzugt für N steht,
dadurch gekennzeichnet, dass man
Alkoxyenone und Enaminoketone der Formel (II) 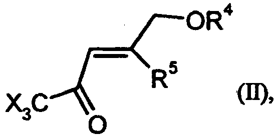 in welcher
- R⁴: für H, Alkyl, Arylalkyl, -(C=O)Alkyl, (C=O)Halogenalkyl, -(C=O)O-Alkyl, SO₂Alkyl, SO₂ - Halogenalkyl, SO₂-Aryl steht,
- X: für Fluor, Chlor steht,
- R⁵: für Alkoxy, Dialkylamino, Cycloalkylamino, Thioalkyl, für Cycloalkyl, welches gegebenenfalls 1-3 Heteroatome aus der Reihe O,N,S enthalten kann, steht,
mit Arylhydrazinen der Formel (III) 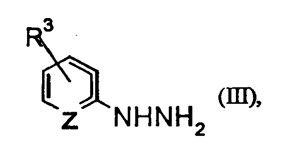 in welcher
- R³: for H, Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
- Z: für CH, N steht,
umsetzt zu 1-Aryl-substituierten dihydro-1H-Pyrazolen der Formel (IV), 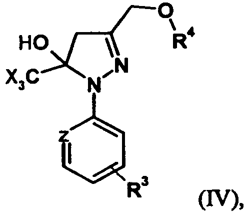 in welcher X, R³, R⁴, Z die oben angegebenen Bedeutungen haben,
diese gegebenenfalls ohne vorherige Isolierung unter Wasserabspaltung weiter umsetzt zu 1-Aryl-substituierten-Trihalogenmethylpyrazolen der Formel (V) 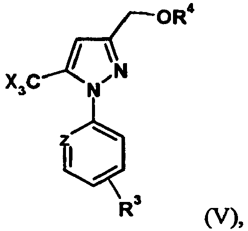 in welcher X, R³, R⁴, Z die oben angegebenen Bedeutungen haben,
diese Verbindungen der allgemeinen Formel (V)
unter Zugabe von beispielsweise HCl, H₂SO₄ oder einer Base umsetzt zu Pyrazolcarbonsäuren der Formel (VI), 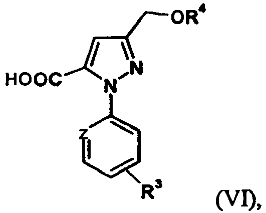 in welcher R³, R⁴, Z die oben angegebenen Bedeutungen haben,
diese nach Abspaltung der Gruppe R⁴ zu Hydroxymethylpyrazolsäuren der Formel (VII) umsetzt, 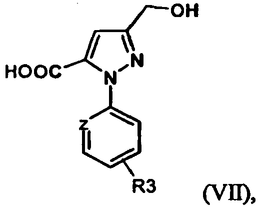 in welcher R³, Z die oben angegebenen Bedeutungen haben,
und diese zu Verbindungen der Formel (I) umsetzt

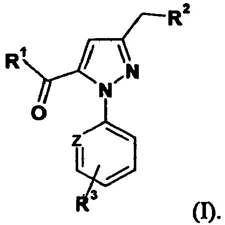

Insbesondere zeichnet sich die erfindungsgemäße Umsetzung durch den Einsatz günstiger Rohstoffe wie Alkoxyalkylen, beispielsweise Alkoxypropen, Säurechloride und Arylhydrazine, sowie durch eine auch im großtechnischen Maßstab besonders gut und einfach durchzuführende Prozeßführung aus.

Das erfindungsgemäße Verfahren kann anhand des folgenden Schemas (I) erläutert werden :

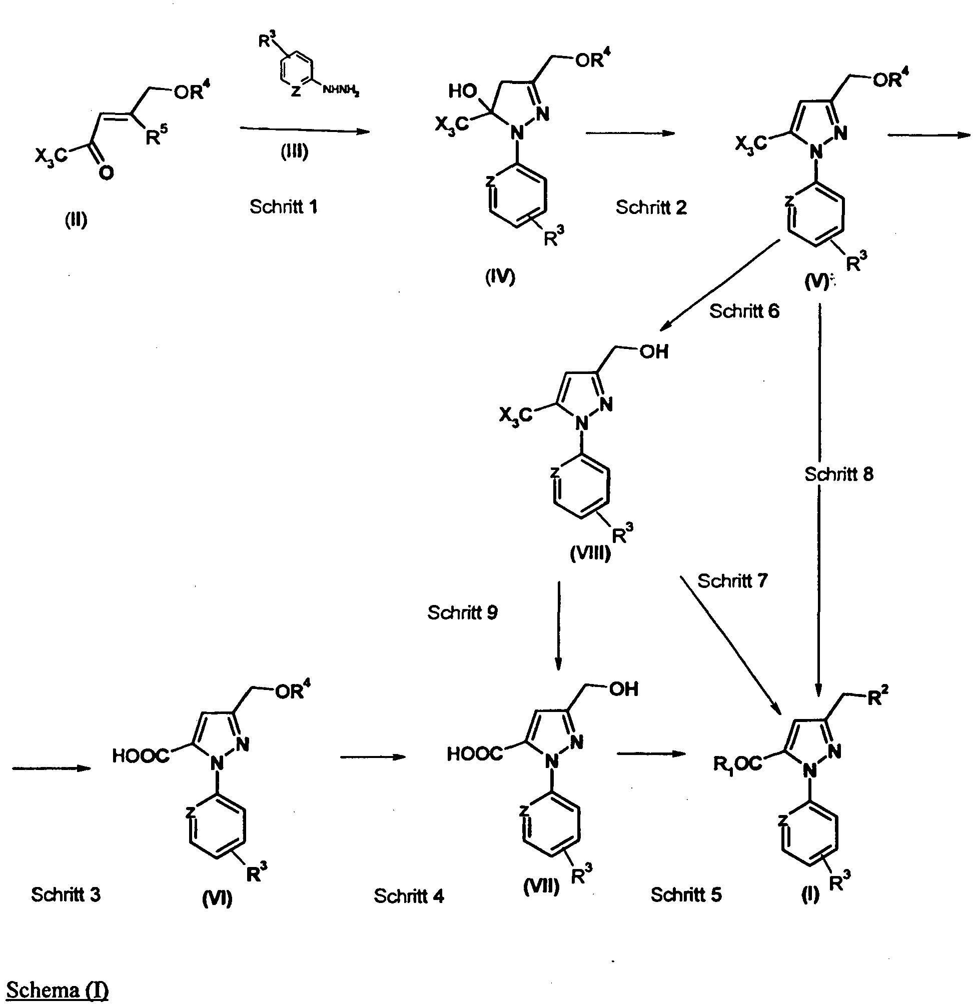

Wobei X, R¹, R², R³, R⁴, R⁵, Z die oben angegebenen Bedeutungen haben.

Die Umsetzung einer Verbindung der Formel (VII) zu einer Verbindung der Formel (I) erfolgt nach üblichen Methoden und wird beispielhaft anhand des nachfolgenden Schemas II erläutert:

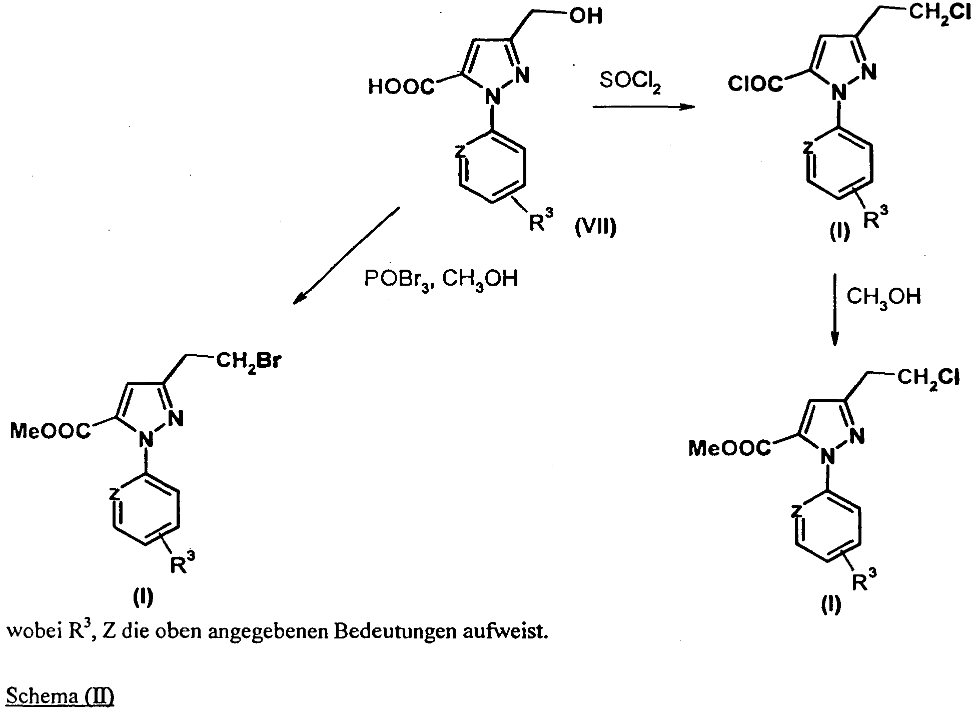

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen = (Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen, welches gegebenenfalls 1-3 Heteroatome aus der Reihe O,N,S enthalten kann, steht,

Aryl-Reste sind im Zusammenhang mit der vorliegenden Erfindung, soweit nicht abweichend definiert, C₆-C₁₀ aromatische Kohlenwasserstoff-Reste und aromatische Kohlenwasserstoff-Reste, die ein, zwei oder mehrere Heteroatome, die ausgewählt sind aus O, N, P und S aufweisen können und optional durch weitere Gruppen substituiert sein können.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. AlkoxyGruppen, die eine Alkylenkette aufweisen können. Im Einzelnen umfasst die Definition Arylalkyl beispielsweise die Bedeutungen Benzyl- und Phenylethyl-; die Definition Arylalkoxy bespielsweise die Bedeutung Benzyloxy.

Alkylaryl-Gruppen (Alkaryl-Gruppen) und Alkylaryloxy-Gruppen sind im Zusammenhang mit der vorliegenden Erfindung, soweit nicht abweichend definiert, durch Alkyl-Gruppen substituierte Aryl-Gruppen, bzw Aryloxy-Gruppen, die eine C₁₋₈Alkylenkette aufweisen können und im Arylgerüst oder Aryloxygerüst ein oder mehrere Heteroatome, die ausgewählt sind aus O, N, P und S, aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

### Alkoxyenones und Enaminoketones der Formel (II)

Die bei der Durchführung des erfindungsgemäßen Verfahrens als Ausgangsstoffe verwendeten Enones sind durch die Formel (II) allgemein definiert. 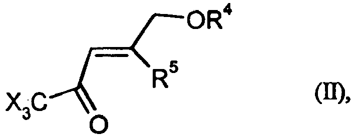 wobei X für Fluor, Chlor, besonders bevorzugt für Chlor steht,
- R⁴: für H, Alkyl, Arylalkyl, (C=O)Alkyl, (C=O)Halogenalkyl,-(C=O)O-Alkyl, SO₂Alkyl, SO₂-Halogenalkyl, SO₂-Aryl steht,
- R⁴: bevorzugt für Aryl(C₁-C₆)-alkyl, (C=O)(C₁-C₆)-Alkyl, (C=O)Halogen(C₁-C₆)-alkyl, -(C=O)O-(C₁-C₆)-Alkyl, SO₂(C₁-C₆)-Alkyl, SO₂Phenyl, SO₂-Halogen(C₁-C₆)-alkyl steht,
- R⁴: besonders bevorzugt für (C=O)(C₁-C₆)-Alkyl, (C=O)Halogen(C₁-C₆)-alkyl, -(C=O)O-(C₁-C₆)-Alkyl, SO₂(C₁-C₆)-Alkyl steht,
- R⁴: ganz besonders bevorzugt für (C=O)(C₁-C₆)-Alkyl, (C=O)Halogen(C₁-C₆)-alkyl steht,
- R⁴: insbesondere bevorzugt für (C=O)CH₃ steht,
- R⁵: für Alkoxy, Dialkylamino, Cycloalkylamino, Thioalkyl, für Cycloalkyl, welches gegebenenfalls 1-3 Heteroatome aus der Reihe O,N,S enthalten kann, steht,
- R⁵: bevorzugt für (C₁-C₆)-Alkoxy, Di(C₁-C₆)-alkylamino, Morpholino, Thioalkyl steht,
- R⁵: besonders bevorzugt für (C₁-C₄)-Alkoxy steht,
- R⁵: ganz besonders bevorzugt für Methoxy steht.

Beispiele für erfindungsgemäß geeignete Alkoxyenone und Enaminoketone der Formel (II) sind z.B.

5,5,5-Trichloro-2-methoxy-4-oxopent-2-en-1-yl acetate, 1,1,1-Trichloro-5-hydroxy-4-methoxypent-3-en-2-one, 5-(Benzyloxy)-1,1,1-trichloro-4-methoxypent-3-en-2-one, 5,5,5-Trifluor-2-methoxy-4-oxopent-2-en-1-yl acetate, (2Z)-5,5,5-trichloro-2-methoxy-4-oxopent-2-en-1-yl trichloroacetate.

Die Verbindungen der Formel (II) sind neu mit Ausnahme derjenigen Verbindung der Formel (II), worin X für Cl, R⁴ für Phenyl und R⁵ für Methoxy steht, welche bereits in der Literatur beschrieben wurde(vgl.Synthesis 2001, N. 13, 1959).

Die Verbindungen der Formel (II) können beispielweise hergestellt werden, indem man 5-Bromo-1,1,1-trihalo-4-alkoxypent-3-en-2-one mit geeigneten O-Nukleophilen unter bestimmten Reaktionsbedingungen umsetzt.

### Arylhydrazine der allgemeinen Formel (III)

Die gemäß der vorliegenden Erfindung verwendeten Hydrazinopyridine sind Verbindungen der allgemeinen Formel (III) 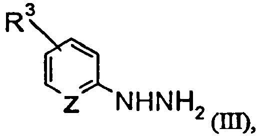 in welcher
- R³: für H, Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
- R³: bevorzugt für H, Halogen, CN, NO₂, (C₁-C₆)-Alkyl, Halogen(C₁-C₆)-alkyl, (C₁-C₆)Alkoxy, Halogen(C₁-C₆)alkoxy, steht,
- R³: besonders bevorzugt für F, Chlor, Brom, Jod, CN, (C₁-C₄)-Alkyl, Halogen(C₁-C₄)-alkyl, Halogen(C₁-C₄)alkoxy, steht,
- R³: ganz besonders bevorzugt für Fluor, Chlor, Brom, Jod steht,
- R³: insbesondere bevorzugt für Chlor steht,
- Z: für CH, N steht,
- Z: bevorzugt und besonders bevorzugt für N steht.

Beispiele für erfindungsgemäß geeignete Hydrazinopyridine sind 3-Chloro-2-hydrazinopyridin, Phenylhydrazin, o- und p-Chlorphenylhydrazin, Nitrophenylhydrazine, O-Methylphenyl-hydrazin.

### Schritt (1)

In einer ersten Ausführungsform des vorliegenden Verfahrens, werden zunächst Alkoxyenone und Enaminoketone der Formel (U) mit Arylhydrazinen der Formel (III) umgesetzt. Im Anschluss daran werden die im Schritt (1) gebildeten Zwischenprodukte zu den 5-Trihalogenmethylpyrazol-Derivaten der Formel (V) unter Wasser Abspaltung umgesetzt (Schritt 2). 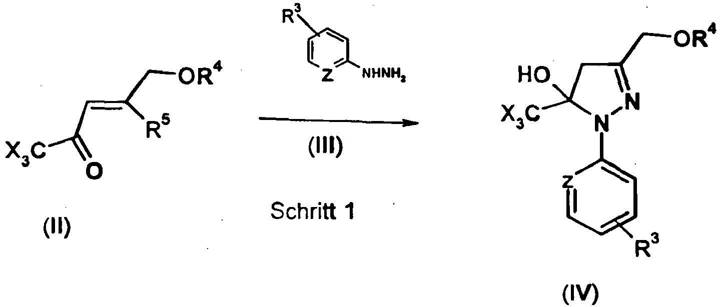 in welcher Z, X, R³, R⁴ und R⁵ die oben angegebenen Bedeutungen haben.

Überraschenderweise würde gefunden, dass die Umsetzung von Alkoxyenonen und Enaminoketonen der Formel (II) mit Arylhydrazinen der Formel (III) regioselektiv erfolgt zu 1-(Aryl)-dihydropyrazololen der Formel (IV). Das zweite Regioisomer wurde nicht beobachtet. Ferner es ist bekannt, dass organische Acetate, Sulphonate oder Carbonate mit N-Nukleophilen (wie z.B Amine oder Hydrazine) unter Abspaltung der Acetat-Gruppe, Sulphonat-Gruppe oder der Carbonat-Gruppe zu Amiden und Hydraziden reagieren. Deswegen es ist als überraschend anzusehen, dass im Falle der Reaktion von Alkoxyenonen der Formel (II), wobei R⁴ für (C=O)Alkyl, (C=O)Halogenalkyl,-(C=O)O-Alkyl, SO₂Alkyl, SO₂-Halogenalkyl, SO₂-Aryl steht, mit Arylhydrazinen der allgemeinen Formel (III), welche starke Nukleophilie aufweisen, nur die Cyclisierung stattfindet und bis dato unbekannte Aryl-5-hydroxy-5-(haloalkyl)-4,5-dihydro-1H-pyrazol-3-yl derivate der Formel (IV) in hoher Ausbeute entstehen.

Die Durchführung des erfindungsgemäßen Verfahrensschritts (1) erfolgt vorzugsweise innerhalb eines Temperaturbereichs von -20°C bis +100°C, besonders bevorzugt bei Temperaturen von -10°C bis +80 °C.

Der erfindungsgemäße Verfahrensschritt (1) wird im Allgemeinen unter Normaldruck durchgeführt. Alternativ ist es jedoch auch möglich, im Vakuum zu arbeiten, um das Wasser und Alkohol zu entfernen.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol des Enones der Formel (II) mit 0.8 Mol bis 1.5 Mol, vorzugsweise 0.9 Mol bis 1.2 Mol, besonders bevorzugt mit der äquimolaren Menge des Arylhydrazines der Formel (III) um.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol. Besonders bevorzugt verwendet man Toluol, Ethanol, Methyltert.Butylether THF, Acetonitril. Die gebildeten Aryl-5-hydroxy-5-(haloalkyl)-4,5-dihydro-1H-pyrazol-3-yl-Derivaten können ohne vorherige Aufarbeitung im darauffolgenden Schritt (2), in welchem Wasserabspaltung stattfindet, eingesetzt werden. In manchen Fällen findet die Wasserabspaltung schon während der Cyclisierung statt.

Alternativ können diese Zwischenprodukte durch geeignete Aufarbeitungsschritte und ggf. weitere Aufreinigung isoliert werden. Erst zu einem späteren Zeitpunkt kann dann Wasser abgespalten werden.

### Schritt 2. Wasserabspaltung

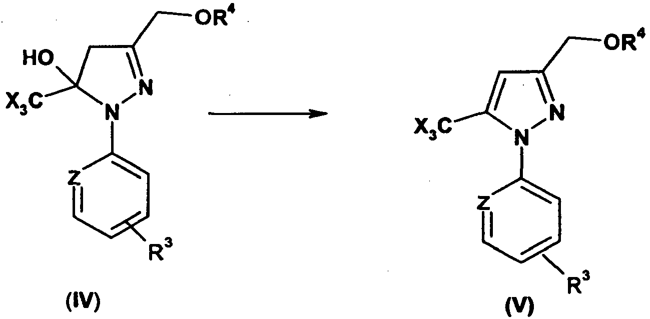

in welcher Z, X, R³, R⁴ die oben angegebenen Bedeutungen haben.

Für die Wasserabspaltung kommen folgende Reagenzien in Frage: H₂SO₄, CF₃COOH, PivCl, POCl₃, Polyphosphorsäure, SOCl₂, (CH₃CO)₂O, (CF₃CO)₂O, Oxalylchlorid, Phosgen und Diphosgen.

Bevorzugt sind (CF₃CO)₂O, Thionylchlorid, Oxalylchlorid und Phosgen.

Die Durchführung des erfindungsgemäßen Verfahrensschritts (A) erfolgt vorzugsweise innerhalb eines Temperaturbereichs von -20°C bis +100°C, besonders bevorzugt bei Temperaturen von -10°C bis +70 °C.

Der erfindungsgemäße Verfahrensschritt (2) wird im Allgemeinen unter Normaldruck durchgeführt. Alternativ ist es jedoch auch möglich, im Vakuum oder unter Überdrück (z.B. Umsetzung mit Phosgen) zu arbeiten. Es ist auch möglich das Wasser nur thermisch abzuspalten.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol des 1-(Aryl)-5-(trihaloalkyl)-4,5-dihydro-1H-pyrazol-5-ol der Formel (IV) mit 0.9 Mol bis 2.5 Mol, vorzugsweise 1 Mol bis 1.8 Mol, besonders bevorzugt mit der äquimolaren Menge des Entwässerungsmittels um.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan. Besonders bevorzugt verwendet man Methyl-tert-butylether, Toluol, Xylol, Dichlorethan, Dichlormethan, Chlorbenzol, Cyclohexan oder Methylcyclohexan, ganz besonders bevorzugt Toluol, Xylol, THF, CH₂Cl₂, Dichlorethan, Methyl-tert-butylether.

### Schritte 3 und 4

In einer bevorzugten Ausführungsform des erfindungsgemäßen Verfahrens wird das 1-Aryl-5-(trihalomethyl)-1H-pyrazol der Formel (V) direkt zur 3-(Hydroxymethyl)-1-(pyridin-2-yl)-1H-pyrazole-5-carbonsäure der Formel (VII) umgewandelt. 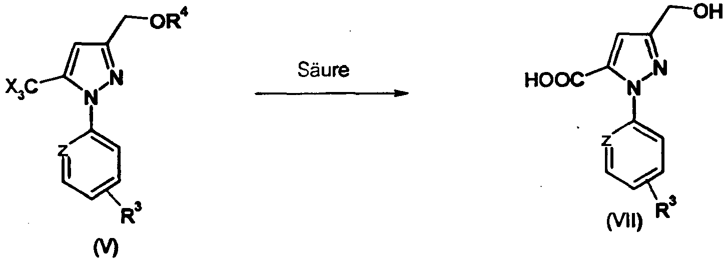 in welcher Z, X, R³, R⁴ die oben angegebenen Bedeutungen haben.

Die Reaktion wird in der Regel unter sauren oder basischen Bedingungen durchgeführt.

Bevorzugt sind mineralische Säuren, beispielsweise H₂SO₄, HCl, HSO₃Cl, HF, HBr, HI, H₃PO₄ oder organische Säuren, beispielsweise CF₃COOH, p-Toluolsulfonsäure, Methansulfonsäure, Trifluormethansulfonsäure.

Die Reaktion kann durch der Zusatz von Katalysatoren wie beispielsweise FeCl₃, AlCl₃, BF₃, SbCl₃, NaH₂PO₄ beschleunigt werden. Die Reaktion kann ohne Zusatz von Säure nur in Wasser durchgeführt werden.

Basische Hydrolyse erfolgt in Gegenwart von organischen Basen wie Trialkylamine, Alkylpyridine, Phosphazene und 1,8-Diazabicyclo[5.4.0]undecen (DBU), anorganischen Basen wie, Alkalimetallhydroxide wie z.B. Lithium-, Natrium- oder Kaliumhydroxid, Alkalimetallalcarbonate (Na₂CO₃, K₂CO₃) und Acetate wie NaOAc, KOAc, LiOAc, -Alkoholate, wie z.B. NaOMe, NaOEt, NaOt-Bu, KOt-Bu.

Die Durchführung des erfindungsgemäßen Verfahrensschritts (A) erfolgt vorzugsweise innerhalb eines Temperaturbereichs von 20°C bis 120°C, besonders bevorzugt bei Temperaturen von 30°C bis 110 °C.

Der erfindungsgemäße Verfahrensschritt (2) wird im Allgemeinen unter Normaldruck durchgeführt. Alternativ ist es jedoch auch möglich, im Vakuum oder unter Überdrück (z.B. Umsetzung im Autoklav mit wässriger HCl) zu arbeiten.

Die Reaktionszeit kann, in Abhängigkeit von der Ansatzgröße und Temperatur, in einem Bereich zwischen 1 Stunde und mehreren Stunden gewählt werden.

### Schritte 6 und 9

In einer weiteren Ausführungsform des erfindungsgemäßen Verfahrens wird zuerst die R⁴ -Gruppe abgespalten (Schritt 6). Anschließend wird die Hydrolyse der Trihalogenmethylgruppe vorgenommen (Schritt 9). 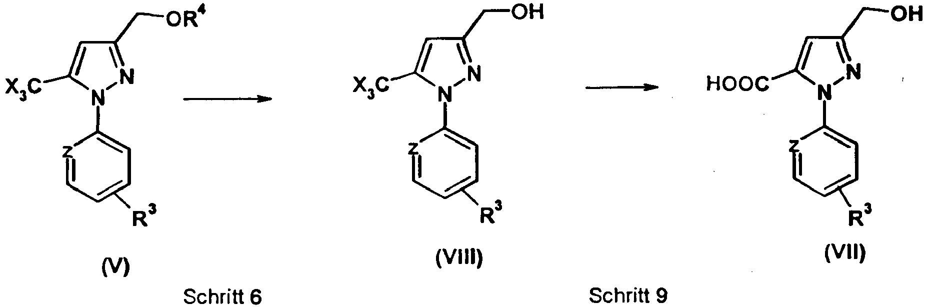 wobei Z, X und R³ die oben angegebenen Bedeutungen haben.

Die Abspaltung der Schutzgruppe ist abhängig von der Definition des Restes R⁴. Falls R⁴ für (C₁-C₆)-Alkyl oder Benzyl steht, kann die Abspaltung in Gegenwart von BBr₃, HCl, HJ, Me₃SiI, PyHCl, FeCl₃, BF₃, im Fall von Benzyl zusätzlich mit Wasserstoff in Gegenwart eines Katalysator erfolgen. Die Abspaltung von Acetat-, Mesylat- oder Sulphonat -Gruppe erfolg in der Regel unter sauren oder basischen Bedingungen. Bevorzugt sind mineralische Säuren, beispielsweise H₂SO₄, HCl, HSO₃Cl, HF, HBr, HI, H₃PO₄ oder organische Säuren, beispielsweise CF₃COOH, p-Toluolsulfonsäure, Methansulfonsäure, Trifluormethansulfonsäure.

Die Abspaltung kann auch ohne Zusatz von Säuren oder Basen bei erhitzen in Wasser durchgeführt werden.

Basische Hydrolyse erfolg in der Regel mit billigen anoarganischen Basen wie Alkalimetallhydroxide z.B. Lithium-, Natrium- oder Kaliumhydroxid, Alkalimetallalcarbonate (Na₂CO₃, K₂CO₃) und Acetate wie NaOAc, KOAc, LiOAc, -Alkoholate, wie z.B. NaOMe, NaOEt, NaOt-Bu, KOt-Bu. Organische Basen wie Trialkylamine, Alkylpyridine, Phosphazene und 1,8-Diazabicyclo[5.4.0]undecen (DBU), können auch eingesetzt werden.

### Schritt 8

Falls R⁴ für Alkyl oder Benzyl steht, kann die CX₃-Gruppe direkt zur Estergruppe umgewandelt werden. Somit können Verbindungen der Formel (V) direkt in die Verbindungen der Formel (I) umgewandelt werden (Schritt 8). 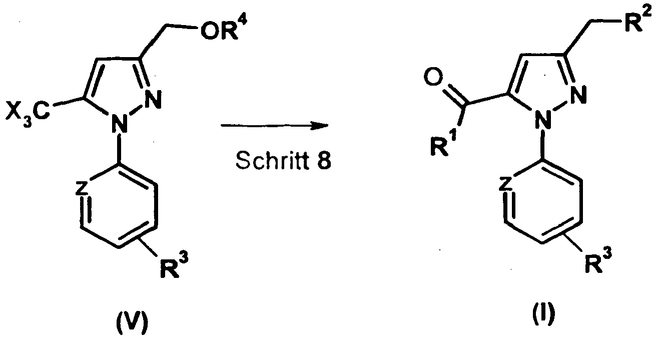 wobei
- X, R³, Z: die oben angegebenen Bedeutungen aufweisen,
- R¹: für (C₁-C₆)-Alkoxy steht,
- R¹: bevorzugt für Methoxy, Ethoxy, Propoxy steht,
- R²: für (C₁-C₆)-Alkoxy, Aryl(C₁-C₆)-alkoxy steht,
- R²: bevorzugt für Aryl (C₁-C₆)-Alkoxy steht.

Für diese Zwecke benutzt man z.B. Alkohole, beispielsweise Methanol, Ethanol, Propanol oder die Kombinationen Alkohol/HCl, Alkohol/FeCl₃, Alkohol/H₂SO₄ oder Alkohol/Alkoholat.

Der Reaktionsschritt 8 kann in Substanz oder in einem Lösungsmittel durchgeführt werden. Vorzugsweise wird die Reaktion in einem Lösungsmittel durchgeführt.Geeignete Lösungsmittel sind beispielsweise ausgewählt aus der Gruppe bestehend aus Wasser, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wir Dimethylformamid (DMF) oder N-methlypyrollidon (NMP) oder Mischungen solcher Lösungsmittel geeignet, wobei Wasser, Acetonitril, Dichlormethan besonders gut geeignet sind.

Falls OR⁴ für O(C=O)Alk, OSO₂Alk steht (Verbindung der Formel (V)), kann die CX₃-Gruppe direkt zur Estergruppe umgewandelt werden. Somit können die Verbindungen der Formel (V) direkt in die Verbindungen der Formel (I) R² = OH umgewandelt werden (Schritt 8).

### Schritt 7

Falls OR⁴ für OH steht (Verbindung der Formel (VIII)), kann die CX₃-Gruppe direkt zur Estergruppe umgewandelt werden. Somit können die Verbindungen der Formel (VIII) direkt in die Verbindungen der Formel (I) R² = OH umgewandelt werden (Schritt 7). Für diese Zwecke benutz man z.B. Alkohole beispielsweise Methanol, Ethanol, Propanol oder die Kombinationen Alkohol/HCl, Alkohol/FeCl₃, Alkohol/H₂SO₄ oder Alkohol/Alkoholat.

### Schritt 5

Die bei der Durchführung des erfindungsgemäßen Verfahrens verwendeten Verbindungen der Formel (VII) werden in einem zweistufigen Prozess in die Verbindungen der Formel (I) umgewandelt.

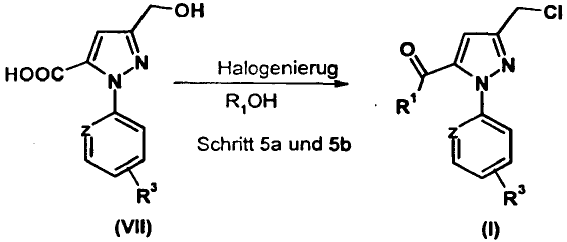

Zuerst werden die Verbindungen der Formel (VII) mit einem Halogenierungsmittel zu den entsprechenden Säurehalogeniden umgewandelt. Gleichzeitig findet auch der Austausch der Hydroxy-Gruppe durch Halogen statt. 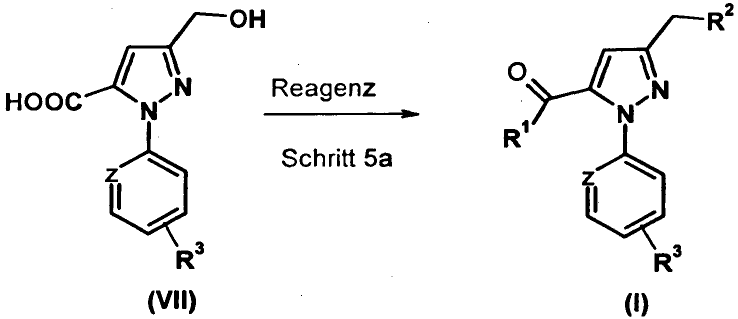 in welcher R¹ für Halogen und R² für Chlor, Brom, Fluor steht.

Zur Bildung der Säurehalogeniden und zum Austausch von Hydroxy gegen Halogen sind folgende Reagenzien geeignet: SOCl₂, POCl₃, Oxalylchlorid, Phosgen, Diphosgen, POBr₃, PBr₃, SF₄, HCF₂CF₂N(Me)₂, PI₃. Bevorzugt sind SOCl₂, Oxalylchlorid, POCl₃, Phosgen.

Die Durchführung des erfindungsgemäßen Halogenierungssschritts (Schritt 5a) erfolgt vorzugsweise innerhalb eines Temperaturbereichs von -20°C bis +100°C, besonders bevorzugt bei Temperaturen von -10°C bis +70 °C.

Der erfindungsgemäße Verfahrensschritt wird im Allgemeinen unter Normaldruck durchgeführt. Alternativ ist es jedoch auch möglich, im Vakuum oder unter Überdrück (z.B. Umsetzung mit Phosgen) zu arbeiten.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol der Säure der Formel (VII) mit 1.9 Mol bis 2.5 Mol, vorzugsweise 1:95 Mol bis 2.2 Mol, besonders bevorzugt mit der äquimolaren Menge (2 Eq) des Chlorierungssmittels um.

Geeignete Lösungsmittel sind beispielsweise aliphatische, alicyclische oder aromatische.Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid;. Besonders bevorzugt verwendet man Toluol, Xylol, Chlorbenzol, n-Hexan, Cyclohexan oder Methylcyclohexan" Methylenchlorid, Dichlorethan, ganz besonders bevorzugt Toluol, Xylol, CH₂Cl₂, ClCH₂CH₂Cl.

Im **Schritt** 5b reagieren die Säurehalogenide mit Alkohol unter Bildung von Estern der Formel (I).

Bevorzugt sind die Alkohole wie : Methanol, Ethanol, Propanol, i-Propanol, Cyclohexanol.

Die Durchführung des erfindungsgemäßen Verfahrensschritts erfolgt vorzugsweise innerhalb eines Temperaturbereichs von -20°C bis +100°C, besonders bevorzugt bei Temperaturen von -10°C bis +40 °C.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol des Säurehalogenids der Formel (VII) mit 1 bis 3 Eq, bevorzug 1 Eq. des Alkohols um. Die Reaktion kann in Alkohol als Lösemitteln durchgeführt werden. Die Halogenierung und Umsetzung mit Alkohol wird in der Regel als Eintopfreaktion durchgeführt.

Die erfindungsgemäßen Verbindungen der Formel (I) werden sind wertvolle Zwischenprodukte in der Synthese von Anthranilsäureamiden (W02007/112893, W02007/144100).

### Herstellbeispiele

### Beispiel 1

*[5-Bromo-1,1,1-trichloro-4-methoxypent-3-en-2-one* wurde hergestellt nach der Methode von Gerus et al., Synthesis 2001, N. 3, 431-436. Ausbeute 90 %.

### Beispiel 2

### 5,5,5-Trichloro-2-methoxy-4-oxopent-2-en-1-yl acetate.

29.6 g (0.1 mol) 5-Bromo-1,1,1-trichloro-4-methoxypent-3-en-2-one, 17 g Kaliumacetate, 5 g Tetrabutylammoniumbromid und 8 g Essigsäure wurden in 300 ml Acetonitril 16 Std. bei 40°C gerührt. Das Gemisch wurde im Vakuum aufkonzentriert und zu dem Rückstand wurde das Wasser zugegeben. Das Produkt wurde mit Ethylacetat extrahiert, die organische Phase mit Wasser gewaschen und das Lösemittel im Vakuum vollständig entfernt.

Man erhielt 25.4 g (85%) des Produktes als hell-braunes Feststoff mit der LC Reinheit von 97 %, Schmp. 53-55°C.

¹H NMR (DMSO d₆) δ: 2.05 (s, 3H), 3.85 (s, 3H), 5.2 (s, 2H), 6.1 (s, 1H) ppm. GC/MS m/Z 275.

### Beispiel 3

### [1-(3-Chloropyridin-2 yl)-5-hydroxy-5-(trichloromethyl)-4,5-dihydro-1H-pyrazol-3-yl]methyl acetate.

27.5 g (0.1 mol) 5,5,5-Trichloro-2-methoxy-4-oxopent-2-en-1-yl acetate und 14.4 g (0.1 mol) 3-Chlor-2-hydrazinopyridine wurden in 200 ml Ethanol vorgelegt und das Gemisch 3 Std. bei 25°C gerührt. Der Niederschlag wurde abfiltriert und mit Cyclohexan gewaschen.

Man erhielt 34 g des Produktes (90 % Ausbeute) als weißer Feststoff mit einem Schmelzpunkt von 105-106°C.

¹H NMR(DMSO d₆) δ: 2.07 (s, 3H), 3.30 (dt, 1H), 3.78 (dt, 1H), 4.79 (dt, 1H), 4.84 (dt, 1H), 7.23 (dd, 1H), 7.95 (dd, 1H), 8.22 (dd, 1H), 9.46 (br.s, 1H) ppm.

### Beispiel 4

### 1-(3-Chloropyridin-2-yl)-5-(trichloromethyl)-1H-pyrazol-3-yl]methyl acetate.

38.7 g von [1-(3-Chloropyridin-2-yl)-5-hydroxy-5-(trichloromethyl)-4,5-dihydro-1H-pyrazol-3-yl]methyl acetate wurden in 200 ml Methyltert-butylether gelöst und 12.6 g von Oxalylchlorid wurden binnen 2 Std. zugetropft (starke Gasentwicklung).

Das Gemisch wurde 5 Std. bei 25°C nachgerührt und im Vakuum komplett eingeengt.

Man erhielt 36 g des Produktes als zähes Öl, welches nach ca. 10 Std bei Raumtemperatur durchkristallisiert. Schmp. 40°C.

¹H NMR (DMSO d ₆) δ : 2.0 (s, 3H), 5.1 (dd, 2H), 7.0 (s, 1H), 7.6 (dd,1H), 8.1 (dd, 1H), 8.5 (dd, 1H)ppm.

### Beispiel 5

### [1-(3-Chloropyridin-2-yl)-5-(trichloromethyl)-1H-pyrazol-3-yl]methanol

36.9 g 1-(3-Chloropyridin-2-yl)-5-(trichloromethyl)-1H-pyrazol-3-yl]methyl acetate wurden in 100 ml Ethanol gelöst und 20 g NaOH (als 40 % Lösung in Wasser) wurden zugegeben. Nach 1 Std. wurde das Gemisch mit 300 ml Wasser verdünnt, das Produkt wurde abfiltriert, mit Wasser gewaschen und getrocknet.

Man erhielt 30 g (95 %) des Produktes als weißer Feststoff.

Schmp. 109-111°C.

¹H NMR (DMSO d₆) δ : 4.55 (2H); 6.95 (1H); 7.55 (dd, 1H); 8.05 (dd, 1H); 8.5 (dd, 1H) ppm.

### Beispiel 6

### Hydrochlorid von 1-(3-chloropyridin-2-yl)-3-(hydroxymethyl)-1H-pyrazole-5-carboxylic acid

38.7 g (0.1 mol) von [1-(3-Chloropyridin-2-yl)-5-(trichloromethyl)-1H-pyrazol-3-yl]methanol und 10 g von H₂SO₄ (als 10 % Lösung in Wasser) wurden 3 Std bei 80°C gerührt.

Das Gemisch wurde abgekühlt auf 0°C und der Niederschlag wurde abfiltriert und mit Acetonitril gewaschen und getrocknet.

Ausbeute 90 %. Schmp. 173-175 °C.

¹H NMR (DMSO d₆) δ: 3,5 (b.s, 1H) 4.50, (2H); 5.2 (b. s ), 6.95 (1H); 7.55 (dd, 1H); 8.05 (dd, 1H); 8.5 (dd, 1H), 13 (b.s) ppm.

### Beispiel 7

### 2-[5-carboxy-3-(hydroxymethyl)-1H-pyrazol-1-yl]-3-chloropyridiniumhydrogen chloride

Man arbeitet wie in Beispiel 6 beschrieben nimmt man jedoch

### 1-(3-Chloropyridin-2-yl)-5-(trichlormethyl)-1H-pyrazol-3-yl]methyl acetate.

Ausbeute 95 %. Schmp. 173-175°C.

### Beispiel 8

### 3-[(Benzyloxy)methyl]-1-(3-chloropyridin-2-yl)-1H-pyrazole-5-carboxylic acid

4.4 g von 2-{3-[(Benzyloxy)methyl]-5-(trichloromethyl)-1H-pyrazol-1-yl}-3-chloropyridine und 30 ml 20 % H₂SO₄ wurden 24 Std bei 100°C erhitzt.

Der Niederschlag wurde abfiltriert und mit Wasser gewaschen. Die Ausbeute betrug 92 %.

¹H NMR (CDCl3) δ: 4.61 (2H, s); 4.63 (m, 2H), 6.97 (1H, s); 7.2-7.4 (5H, m); 7.42 (1H,m); 7.96 [(1H,d 2 Hz.)]; 8.5 [(1H, d, 2 Hz)] ppm.

### Beispiel 9

### 1-(3-chloropyridin-2-yl)-3-(hydroxymethyl)-1H-pyrazole-5-carboxylic acid hydrochlorid

3.43 g von 3-[(Benzyloxy)methyl]-1-(3-chloropyridin-2-yl)-1H-pyrazole-5-carboxylic acid und 20 ml HCl (37,5 %) wurden 2 Std bei 100°C erhitzt und dann das Reaktionsgemisch in Vakuum bei 10 mbar komplett eingeengt. Man erhielt 1-(3-chloropyridin-2-yl)-3-(hydroxymethyl)-1H-pyrazole-5-carboxylic acid als Hydrochloridsalz. Durch Neutrallization mit NaHCO₃ wurde die freie Säure als weißer Feststoff erhalten. Die Ausbeute betrug 94 %.

### Beispiel 10.

### Methyl 3-(chlormethyl)-1-(3-chloropyridin-2-yl)-1H-pyrazole-5-carboxylate

1-(3-chloropyridin-2-yl)-3-(hydroxymethyl)-1H-pyrazole-5-carboxylic acid hydrochlorid 29 g (0.1 mol) wurde in 100 ml Toluol vorgelegt. 24 g von SOCl₂ wurden portionsweise bei 60°C zugegeben. Man erhitzte das Gemisch 3 Std. bei 70°C, wobei der Niederschlag komplett in die Lösung ging. Methanol (30 ml) wurde langsam zu dem Gemisch zugetropft und die Lösung 1 Stunde bei 30°C nachgerührt. Anschließend wurde die Lösung im Vakuum eingeengt. Man erhielt 95% des Produktes mit einer Reinheit von 96 %.

¹H NMR (CDCl3) δ: 3.7 (3H,s); 4.7 (2H, s); 7.1 (1H, s); 7.5 (1H, m); 8.05 [(1H, m)]; 8.5 [(1H, m)] ppm

### Beispiel 11.

### Methyl 1-(2-methylphenyl)-3-(hydroxymethyl)-1H-pyrazole-5-carboxylate

30.5 g [1-(2-methylphenyl)-5-(trichloromethyl)-1H-pyrazol-3-yl]methanol und 300 ml Methanol wurden 3 Std. bei 90°C im Autoklaven erhitzt. Methanol wurde im Vakuum entfernt und das Produkt mittels Chromatographie gereinigt. Ausbeute 80%.

### Analytische Charakterisierung

¹H NMR (CD₃CN) δ: 7.4-7.2 (4H, m); 6.95 (1H, s), 4,55 (2H, s); 3,75 (3H,s); 11.95 (3H, s) ppm.

### Beispiel 12.

### Methyl 1-(3-chloropyridin-2-yl)-3-(hydroxymethyl)-1H-pyrazole-5-carboxylate

32.6 g [1-(3-chloropyridin-2-yl)-5-(trichloromethyl)-1H-pyrazol-3-yl]methanol und 300 ml Methanol wurden 3 Std. bei 90°C im Autoklaven erhitzt. Methanol wurde im Vakuum entfernt und der Niederschlag mit Wasser gewaschen. Ausbeute 25 g., 94%. Schmp. 104°C

### Beispiel 13.

### Methyl 3-(chloromethyl)-1-(3-chloropyridin-2-yl)-1H-pyrazole-5-carboxylate

Methyl 1-(3-chloropyridin-2-yl)-3-(hydroxymethyl)-1H-pyrazole-5-carboxylate (26.7 g, 0.1 mol) wurde in 150 ml CH₂Cl₂ gelöst und die Lösung auf 5°C gekühlt. SOCl₂ (0.12 mol) in 30 ml CH₂Cl₂ wurde langsam bei dieser Temperatur zugetropft. Das Gemisch wurde 4 Std. bei 40°C nachgerührt und im Vakuum eingeengt. Das Produkt kann ohne Reinigung weiter eingesetzt werden.

### Analytische Charakterisierung

¹H NMR (CD₃CN) δ: 8,52 (1H,d); 8,06 (1H,d), 7,55 (1H, dd); 7,10 (1H, s); 4,75 (2H,s); 3,75 (3H,s) ppm.

## Patentansprüche

1. Verfahren zur Herstellung von Aryl-substituierten Pyrazol-Derivaten der allgemeinen Formel (I), 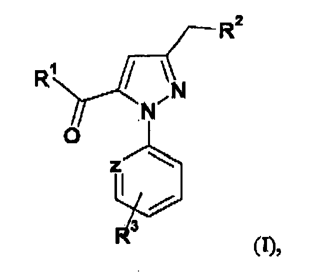 in welcher
R¹ für Hydroxy, Alkoxy, Aryloxy, Halogen steht,
R² für Hydroxy, Alkoxy, Arylalkoxy, Alkylthio, Halogen, O-(C=O)Alkyl, O-(C=O)O-Alkyl, (C=O)Halogenalkyl, OSO₂Alkyl, OSO₂Halogenalkyl, OSO₂-Aryl steht,
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
Z für CH, N steht,
**dadurch gekennzeichnet, dass** man
(A) Alkoxyenone und Enaminoketone der Formel (II) 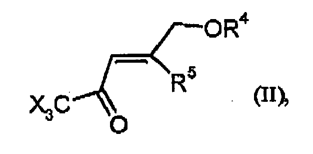 in welcher
R⁴ für H, Alkyl, Arylalkyl, -(C=O)Alkyl, (C=O)Halogenalkyl, -(C=O)O-Alkyl, SO₂Alkyl, SO₂ -Halogenalkyl, SO₂-Aryl steht,
X für Fluor, Chlor, steht,
R⁵ für Alkoxy, Dialkylamino, Cycloalkylamino, Thioalkyl, für Cycloalkyl, welches gegebenenfalls 1-3 Heteroatome aus der Reihe O,N,S enthalten kann, steht,
mit Arylhydrazinen der Formel (III) 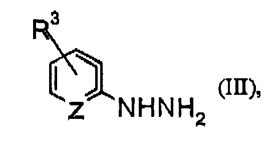 in welcher
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
Z für CH, N steht,
umsetzt zu 1-Aryl-substituierten dihydro-1H-Pyrazolen der Formel (IV), 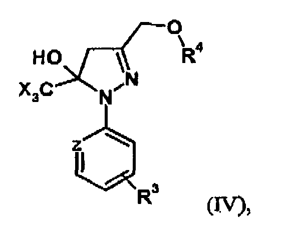 in welcher X, R³, R⁴, Z die oben angegebenen Bedeutungen haben,
(B) diese gegebenenfalls ohne vorherige Isolierung unter Wasserabspaltung weiter umsetzt zu 1-Aryl- substituierten -Trihalogenmethylpyrazolen der Formel (V) 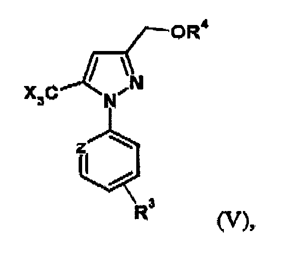 in welcher X, R³, R⁴, Z die oben angegebenen Bedeutungen haben,
(C) diese Verbindungen der allgemeinen Formel (V)
unter Zugabe von beispielsweise HCl, H₂SO₄ oder einer Base umsetzt zu Pyrazolcarbonsäuren der Formel (VI), 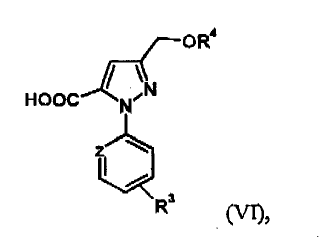 in welcher R³, R⁴, Z die oben angegebenen Bedeutungen haben,
(D) diese nach Abspaltung der Gruppe R⁴ zu Hydroxymethylpyrazolsäuren der Formel (VII) umsetzt, 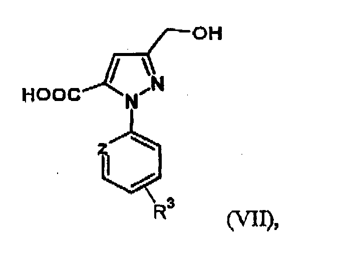 in welcher R³, Z die oben angegebenen Bedeutungen hat, und
(E) diese zu Verbindungen der Formel (I) umsetzt

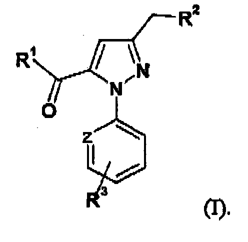

2. Verfahren gemäß Anspruch 1, **dadurch gekennzeichnet, dass**
R¹ für Hydroxy, (C₁-C₆)Alkoxy, Halogen steht,
R² für Hydroxy, Halogen, O-(C=O)(C₁-C₆)Alkyl steht,
R³ für Halogen, CN, NO₂, (C₁-C₆)-Alkyl, Halogen(C₁-C₆)-alkyl, (C₁-C₆)Alkoxy, Halogen(C₁-C₆)alkoxy steht,
X für Fluor, Chlor, steht,
Z für N steht,
R⁴ für Aryl(C₁-C₆)-alkyl, (C=O)(C₁-C₆)-Alkyl, (C=O)Halogen(C₁-C₆)-alkyl, -(C=O)(O-(C₁-C₆)-Alkyl, SO₂(C₁-C₆)-Alkyl, SO₂Phenyl, SO₂-Halogen(C₁-C₆)-alkyl steht,
R⁵ für (C₁-C₆)-Alkoxy, Di(C₁-C₆)-alkylamino, Morpholino, Thioalkyl steht.

3. Verfahren gemäß einem der Ansprüche 1 oder 2, **dadurch gekennzeichnet, dass**
R¹ für (C₁-C₆)Alkoxy, Hydroxy steht,
R² für Hydroxy, C(=O)CH₃ steht
R³ für Chlor steht,
R⁴ für (C=O)CH₃ steht,
R⁵ für Methoxy steht,
X für Chlor steht,
Z für N steht.

4. Verbindungen der allgemeinen Formel (IV) gemäß Anspruch 1, **dadurch gekennzeichnet, dass**
X, R³, Z die oben angegebenen Bedeutungen haben,
R⁴ für (C=O)(C₁-C₆)-Alkyl, (C=O)Halogen(C₁-C₆)-alkyl steht.

5. Verbindungen der allgemeinen Formel (V), gemäß Anspruch 1, **dadurch gekennzeichnet, dass**
X, R³, Z die oben angegebenen Bedeutungen haben,
R⁴ für (C=O)(C₁-C₆)-Alkyl, (C=O)Halogen(C₁-C₆)-alkyl steht.

6. Verbindungen der allgemeinen Formel (IV) gemäß Anspruch 4, **dadurch gekennzeichnet, dass**
R⁴ für (C=O)CH₃ steht und X für Chlor steht.

7. Verbindungen der allgemeinen Formel (V) gemäß Anspruch 5, **dadurch gekennzeichnet, dass**
R⁴ für (C=O)CH₃ steht und X für Chlor steht.

## Claims

1. Process for preparing aryl-substituted pyrazole derivatives of the general formula (I) 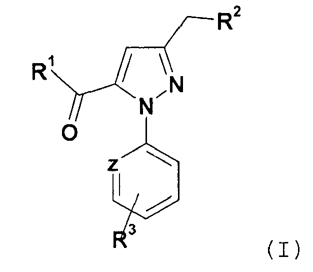 in which
R¹ is hydroxyl, alkoxy, aryloxy, halogen,
R² is hydroxyl, alkoxy, arylalkoxy, alkylthio, halogen, O-(C=O)alkyl, O-(C=O)O-alkyl, (C=O)haloalkyl, OSO₂alkyl, OSO₂-haloalkyl, OSO₂-aryl,
R³ is halogen, CN, NO₂, alkyl, cycloalkyl, haloalkyl, halocycloalkyl, alkoxy, haloalkoxy, alkylamino, dialkylamino, cycloalkylamino,
Z is CH, N,
**characterized in that**
(A) alkoxy enones and enamino ketones of the formula (II) 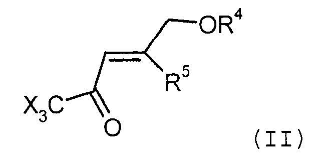 in which
R⁴ is H, alkyl, arylalkyl, -(C=O)alkyl, (C=O)haloalkyl, -(C=O)O-alkyl, SO₂alkyl, SO₂-haloalkyl, SO₂-aryl,
X is fluorine, chlorine,
R⁵ is alkoxy, dialkylamino, cycloalkylamino, thioalkyl, or is cycloalkyl which may optionally contain 1-3 heteroatoms from the group of O, N, S,
are reacted with arylhydrazines of the formula (III) 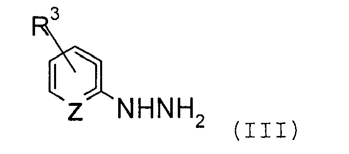 in which
R³ is halogen, CN, NO₂, alkyl, cycloalkyl, haloalkyl, halocycloalkyl, alkoxy, haloalkoxy, alkylamino, dialkylamino, cycloalkylamino,
Z is CH, N,
to give 1-aryl-substituted dihydro-1H-pyrazoles of the formula (IV) 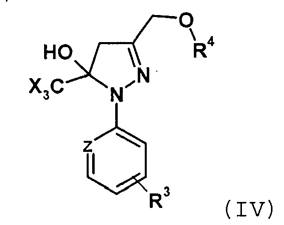 in which X, R³, R⁴, Z are each as defined above,
(B) the latter are optionally converted further, without preceding isolation, with elimination of water, to 1-aryl-substituted trihalomethylpyrazoles of the formula (V) 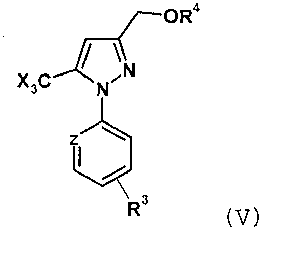 in which X, R³, R⁴, Z are each as defined above,
(C) these compounds of the general formula (V) are converted with addition of HCl, H₂SO₄ or a base, for example, to pyrazolecarboxylic acids of the formula (VI) 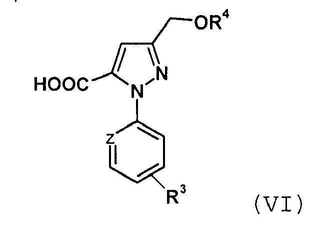 in which R³, R⁴, Z are each as defined above,
(D) the latter are converted, after detaching the R⁴ group, to hydroxymethylpyrazole acids of the formula (VII) 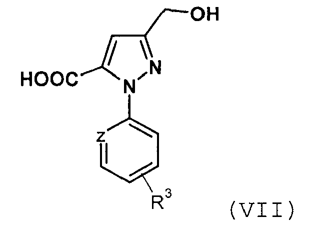 in which R³, Z are as defined above, and
(E) the latter are converted to compounds of the formula (I)

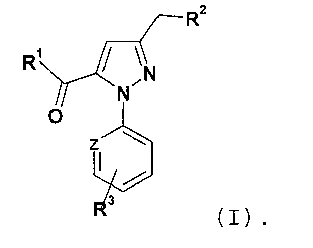

2. Process according to Claim 1, **characterized in that**
R¹ is hydroxyl, (C₁-C₆)-alkoxy, halogen,
R² is hydroxyl, halogen, O-(C=O)(C₁-C₆)alkyl,
R³ is halogen, CN, NO₂, (C₁-C₆)-alkyl, halo(C₁-C₆)-alkyl, (C₁-C₆)-alkoxy, halo(C₁-C₆)alkoxy,
X is fluorine, chlorine,
Z is N,
R⁴ is aryl (C₁-C₆) -alkyl, (C=O)(C₁-C₆) -alkyl, (C=O)halo(C₁-C₆)-alkyl, -(C=O)O-(C₁-C₆)-alkyl, SO₂(C₁-C₆)-alkyl, SO₂phenyl, SO₂ -halo(C₁-C₆)-alkyl,
R⁵ is (C₁-C₆-alkoxy, di(C₁-C₆)-alkylamino, morpholino, thioalkyl.

3. Process according to either of Claims 1 and 2,
**characterized in that**
R¹ is (C₁-C₆)alkoxy, hydroxyl,
R² is hydroxyl, C(=O)CH₃,
R³ is chlorine,
R⁴ is (C=O)CH₃,
R⁵ is methoxy,
X is chlorine,
Z is N.

4. Compounds of the general formula (IV) according to Claim 1, **characterized in that**
X, R³, Z are each as defined above,
R⁴ is (C=O) (C₁-C₆) -alkyl, (C=O)halo(C₁-C₆) -alkyl.

5. Compounds of the general formula (V) according to Claim 1, **characterized in that**
X, R³, Z are each as defined above,
R⁴ is (C=O) (C₁-C₆)-alkyl, (C=O)halo(C₁-C₆)-alkyl.

6. Compounds of the general formula (IV) according to Claim 4, **characterized in that**
R⁴ is (C=O)CH₃ and X is chlorine.

7. Compounds of the general formula (V) according to Claim 5, **characterized in that**
R⁴ is (C=O)CH₃ and X is chlorine.

## Revendications

1. Procédé de fabrication de dérivés de pyrazole à substitution aryle de formule générale (I) 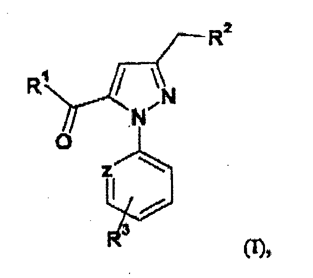 dans laquelle
R¹ représente hydroxy, alcoxy, aryloxy, halogène,
R² représente hydroxy, alcoxy, arylalcoxy, alkylthio, halogène, O-(C=O)-alkyle, O-(C=O)O-alkyle, (C=O)-halogénoalkyle, OSO₂-alkyle, OSO₂-halogénoalkyle, OSO₂-aryle,
R³ représente halogène, CN, NO₂, alkyle, cycloalkyle, halogénoalkyle, halogénocycloalkyle, alcoxy, halogénoalcoxy, alkylamino, dialkylamino, cycloalkylamino,
Z représente CH, N,
**caractérisé en ce que**
(A) des alcoxyénones et des énaminocétones de formule (II) 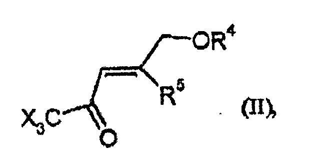 dans laquelle
R⁴ représente H, alkyle, arylalkyle, (C=O) -alkyle, (C=O)-halogénoalkyle, -(C=O)O-alkyle, SO₂-alkyle, SO₂-halogénoalkyle, SO₂-aryle,
X représente fluor, chlore,
R⁵ représente alcoxy, dialkylamino, cycloalkylamino, thioalkyle, cycloalkyle, qui peut éventuellement contenir 1 à 3 hétéroatomes de la série O, N, S,
sont mises en réaction avec des arylhydrazines de formule (III) 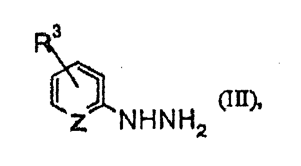 dans laquelle
R³ représente halogène, CN, NO₂, alkyle, cycloalkyle, halogénoalkyle, halogénocycloalkyle, alcoxy, halogénoalcoxy, alkylamino, dialkylamino, cycloalkylamino,
Z représente CH, N,
pour former des dihydro-1H-pyrazoles à substitution 1-aryle de formule (IV) 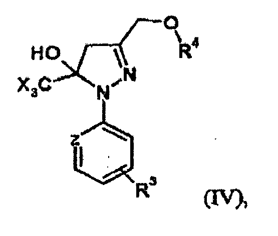 dans laquelle X, R³, R⁴, Z ont les significations indiquées précédemment,
(B) ceux-ci sont ensuite mis en réaction, éventuellement sans isolement préalable, par clivage d'eau, pour former des trihalogénométhylpyrazoles à substitution 1-aryle de formule (V) 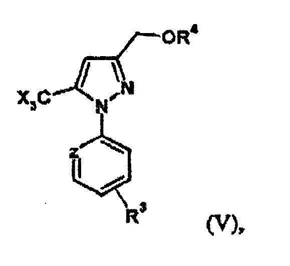 dans laquelle X, R³, R⁴, Z ont les significations indiquées précédemment,
(C) ces composés de formule générale (V) sont mis en réaction avec ajout par exemple d'HCl, H₂SO₄ ou d'une base pour former des acides pyrazolecarboxyliques de formule (VI) 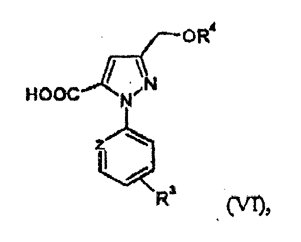 dans laquelle R³, R⁴, Z ont les significations indiquées précédemment,
(D) ceux-ci sont mis en réaction après clivage du groupe R⁴ pour former des acides hydroxyméthylpyrazoliques de formule (VII) 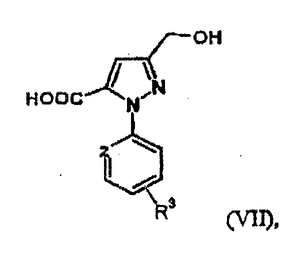 dans laquelle R³, Z ont les significations indiquées précédemment, et
(E) ceux-ci sont transformés en composés de formule (I)

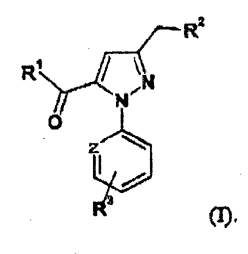

2. Procédé selon la revendication 1, **caractérisé en ce que**
R¹ représente hydroxy, alcoxy en (C₁-C₆), halogène,
R² représente hydroxy, halogène, O-(C=O)-alkyle en (C₁-C₆),
R³ représente halogène, CN, NO₂, alkyle en (C₁-C₆), halogénoalkyle en (C₁-C₆), alcoxy en (C₁-C₆), halogénoalcoxy en (C₁-C₆),
X représente fluor, chlore,
Z représente N,
R⁴ représente arylalkyle en (C₁-C₆), - (C=O) -alkyle en (C₁-C₆), (C=O)-halogénoalkyle en (C₁-C₆), -(C=O)O-alkyle en (C₁-C₆), SO₂-alkyle en (C₁-C₆), SO₂-phényle, SO₂-halogénoalkyle en (C₁-C₆),
R⁵ représente alcoxy en (C₁-C₆), dialkylamino en (C₁-C₆), morpholino, thioalkyle.

3. Procédé selon l'une quelconque des revendications 1 ou 2, **caractérisé en ce que**
R¹ représente alcoxy en (C₁-C₆), hydroxy,
R² représente hydroxy, C(=O)CH₃,
R³ représente chlore,
R⁴ représente (C=O)CH₃,
R⁵ représente méthoxy,
X représente chlore,
Z représente N.

4. Composés de formule générale (IV) selon la revendication 1, **caractérisés en ce que**
X, R³, Z ont les significations indiquées précédemment, R⁴ représente (C=O)-alkyle en (C₁-C₆), (C=O)-halogénoalkyle en (C₁-C₆).

5. Composés de formule générale (V) selon la revendication 1, **caractérisés en ce que**
X, R³, Z ont les significations indiquées précédemment, R⁴ représente (C=O)-alkyle en (C₁-C₆), (C=O)-halogénoalkyle en (C₁-C₆).

6. Composés de formule générale (IV) selon la revendication 4, **caractérisés en ce que**
R⁴ représente (C=O)CH₃ et X représente chlore.

7. Composés de formule générale (V) selon la revendication 5, **caractérisés en ce que**
R⁴ représente (C=O)CH₃ et X représente chlore.
